(19) Europäisches Patentamt
European Patent Office
Office européen des brevets

(11) EP 1 634 965 B1

(12) # EUROPEAN PATENT SPECIFICATION

(45) Date of publication and mention of the grant of the patent:
**20.01.2010 Bulletin 2010/03**

(51) Int Cl.:
*C12Q 1/68* (2006.01) *C12Q 1/42* (2006.01)

(21) Application number: **05019405.9**

(22) Date of filing: **07.09.2005**

(54) **Real time PCR with the addition of pyrophosphatase**

Echtzeit-PCR unter Zusatz von Pyrophosphatase

PCR en temps réel en présence de pyrophosphatase

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.09.2004 EP 04021399**

(43) Date of publication of application:
**15.03.2006 Bulletin 2006/11**

(73) Proprietors:
- **Roche Diagnostics GmbH**
  **68305 Mannheim (DE)**
  Designated Contracting States:
  **DE**
- **F.HOFFMANN-LA ROCHE AG**
  **4070 Basel (CH)**
  Designated Contracting States:
  **AT BE BG CH CY CZ DK EE ES FI FR GB GR HU IE IS IT LI LT LU LV MC NL PL PT RO SE SI SK TR**

(72) Inventors:
- **Boell, Inga**
  **82377 Penzberg (DE)**
- **Degen, Anja**
  **82380 Peissenberg (DE)**
- **Heindl, Dieter**
  **82396 Paehl (DE)**
- **Sagner, Gregor**
  **82377 Penzberg (DE)**

(56) References cited:
- **CHADWICK R B ET AL: "HETEROZYGOTE AND MUTATION DETECTION BY DIRECT AUTOMATED FLUORESCENT DNA SEQUENCEING USING A MUTANT TAQ DNA POLYMERASE" BIOTECHNIQUES, EATON PUBLISHING, NATICK, US, vol. 20, no. 4, April 1996 (1996-04), pages 676-683, XP008037458 ISSN: 0736-6205**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 634 965 B1

## Description

The present invention relates to the field of nucleic acid amplification and monitoring said amplification in real time. More precisely, the present invention provides a solution for performing real time PCR assays characterized in the fluorescent compound which are present in such assays are adequately stabilized.

## Prior art background

Amplification of DNA by polymerase chain reaction (PCR) is a technique fundamental to molecular biology. Nucleic acid analysis by PCR requires sample preparation, amplification, and product analysis. Although these steps are usually performed sequentially, amplification and analysis can occur simultaneously. DNA dyes or fluorescent probes can be added to the PCR mixture before amplification and used to analyze PCR products during amplification. Sample analysis occurs concurrently with amplification in the same tube within the same instrument. This combined approach decreases sample handling, saves time, and greatly reduces the risk of product contamination for subsequent reactions, as there is no need to remove the samples from their closed containers for further analysis. The concept of combining amplification with product analysis has become known as "real time" PCR. See, for example, U.S. Patent No. 6,174,670.

### Real Time PCR detection formats

In kinetic real time PCR, the formation of PCR products is monitored in each cycle of the PCR. The amplification is usually measured in thermocyclers which have additional devices for measuring fluorescence signals during the amplification reaction.

### a) DNA binding dye format

Since the amount of double stranded amplification product usually exceeds the amount of nucleic acid originally present in the sample to be analyzed, double-stranded DNA specific dyes may be used, which upon excitation with an appropriate wavelength show enhanced fluorescence only if they are bound to double-stranded DNA. Preferably, only those dyes may be used which like SybrGreen I, for example, do not affect the efficiency of the PCR reaction.

All other fomats known in the art require the design of a fluorescent labeled Hybridization Probe which only emits fluorescence upon binding to its target nucleic acid.

### b) TaqMan probe

A single-stranded Hybridization Probe is labeled with two components. When the first component is excited with light of a suitable wavelength, the absorbed energy is transferred to the second component, the so-called quencher, according to the principle of fluorescence resonance energy transfer. During the annealing step of the PCR reaction, the hybridization probe binds to the target DNA and is degraded by the 5'-3' exonuclease activity of the Taq Polymerase during the subsequent elongation phase. As a result the excited fluorescent component and the quencher are spatially separated from one another and thus a fluorescence emission of the first component can be measured. TaqMan probe assays are disclosed in detail in US 5,210,015, US 5,538,848, and US 5,487,972. TaqMan hybridization probes and reagent mixtures are disclosed in US 5,804,375.

### c) Molecular Beacons

These hybridization probes are also labeled with a first component and with a quencher, the labels preferably being located at both ends of the probe. As a result of the secondary structure of the probe, both components are in spatial vicinity in solution. After hybridization to the target nucleic acids both components are separated from one another such that after excitation with light of a suitable wavelength the fluorescence emission of the first component can be measured (US 5,118,801).

### d) Single Label Probe (SLP) Format

This detection format consists of a single oligonucleotide labeled with a single fluorescent dye at either the 5'- or 3'-end (WO 02/14555). Two different designs can be used for oligo labeling: G-Quenching Probes and Nitroindole-Dequenching probes. In the G-Quenching embodiment, the fluorescent dye is attached to a C at oligo 5'- or 3'-end. Fluorescence decreases significantly when the probe is hybridized to the target, in case two G's are located on the target strand opposite to C and in position 1 aside of complementary oligonucleotide probe. In the Nitroindole Dequenching

embodiment, the fluorescent dye is attached to Nitroindole at the 5'- or 3'-end of the oligonucleotide. Nitroindole somehow decreases the fluorescent signaling of the free probe. Fluorescence increases when the probe is hybridized to the target DNA due to a dequenching effect.

**e) FRET hybridization probes**

The FRET Hybridization Probe test format is especially useful for all kinds of homogenous hybridization assays (Matthews, J.A., and Kricka, L.J., Anal. Biochem. 169 (1988) 1-25). It is characterized by two single-stranded hybridization probes which are used simultaneously and are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected. Alternatively to monitoring the increase in fluorescence of the FRET acceptor component, it is also possible to monitor fluorescence decrease of the FRET donor component as a quantitative measurement of hybridization event.

In particular, the FRET Hybridization Probe format may be used in real time PCR, in order to detect the amplified target DNA. Among all detection formats known in the art of real time PCR, the FRET-Hybridization Probe format has been proven to be highly sensitive, exact and reliable (WO 97/46707; WO 97/46712; WO 97/46714). As an alternative to the usage of two FRET hybridization probes, it is also possible to use a fluorescent-labeled primer and only one labeled oligonucleotide probe (Bernard, P.S., et al., Anal. Biochem. 255 (1998) 101-107). In this regard, it may be chosen arbitrarily, whether the primer is labeled with the FRET donor or the FRET acceptor compound.

Besides PCR and real time PCR, FRET hybridization probes are used for melting curve analysis. In such an assay, the target nucleic acid is amplified first in a typical PCR reaction with suitable amplification primers. The hybridization probes may already be present during the amplification reaction or added subsequently. After completion of the PCR-reaction, the temperature of the sample is constitutively increased, and fluorescence is detected as long as the hybridization probe was bound to the target DNA. At melting temperature, the hybridization probes are released from their target, and the fluorescent signal is decreasing immediately down to the background level. This decrease is monitored with an appropriate fluorescence versus temperature-time plot such that a first derivative value can be determined, at which the maximum of fluorescence decrease is observed.

All probe based detection fortmats can be "multiplexed". More precisely, in one reaction vessel, multiple targets may become amplified with multpiple pairs of amplification primers and detected with multiple habridization probe. In this case, said multiple probes ares labeled with different detectable fluorescent dyes in order to detect and discriminate the multiple targets which are supposed to be found in the sample.

For multiplex detection with the FRET hybridization probe format, it is possible that Fluorescein or Fluorescein derivatives are used as a FRET donor moiety in combination with different FRET acceptor moieties such as Cy-5, LC-Red-640, or LC-red 705.

A typical example for an instrument capable of performing multiplex real time PCR is the Roche Diagnostics LightCycler (Cat. No.3 531 414 201). It is a fast PCR system enabling kinetic on-line PCR quantification and subsequent analysis of PCR-product melting curves. The optical system of the current LightCycler version 2.0 being commercially available contains one light source, a blue light emitting diode (470 nm LED) and six detection channels. A defined signal threshold is determined for all reactions to be analysed and the number of cycles Cp required to reach this threshold value is determined for the target nucleic acid as well as for the reference nucleic acids such as the standard or housekeeping gene. The absolute or relative copy numbers of the target molecule can be determined on the basis of the Cp values obtained for the target nucleic acid and the reference nucleic acid.

The fluorescence emitted by a sample is separated by a set of dichroic mirrors and filters into different wavelengths that can be recorded in one of the six detection channels. Due to the fluorescent compounds which are available on the market, this allows detection of the double-stranded DNA-binding dye SybrGreenl, dual color detection with the TaqMan Probe format and 4-color detection with the Hybridization Probe(HybProbe) format. Details of the LightCycler system are disclosed in WO 97/46707, WO 97/46712 and WO 97/46714.

However, a major drawback with respect to multiplex assays has been observed. In case multiple amplicons are amplified using multiple primer pairs, a decrease in fluorescence is frequently observed.

Thus, the technical problem underlying the present invention is to provide an improved performance for quantitative real time PCR amplification and subsequent melting curve analysis at late amplification cycles.

## Brief description of the invention

The present invention is based on the surprising observation that the presence of Pyrophosphatase may stabilize fluorescent signalling during a real time PCR experiment even at late cycles.

Thus, in a first aspect, the present disclosure is directed to the use of a Pyrophosphatase to stabilize at least a first fluorescent entity which is present in a composition for amplification and detection of at least a first target nucleic acid during a thermocycling procedure.

In a second aspect, the present invention is directed to a composition for amplifying and detecting a target nucleic acid consisting of

- a thermostable DNA Polymerase
- a mixture of deoxynucleoside-tri-phosphates
- a buffer
- multiple pairs of amplification primers,
- multiple hybridization probes each labeled with a different detectable fluorescent dye, which only emits fluorescence upon binding of the probe to its target nucleic acid wherein said composition additionally comprises a Pyrophosphatase.

Preferably, the Pyrophosphatase is a thermostable Pyrophosphatase.

In this context, the present invention also covers such compositions which further comprise a respective target nucleic acid which is supposed to become amplified.

In a specific embodiment, the composition comprises at least a second hybridization probe labeled with a second fluorescent entity, wherein said first and second hybridization probe together constitute a pair of FRET hybridization probes.

In a third aspect, the present invention is also directed to a method for amplifying and detecting at least a first target nucleic acid comprising

- providing a composition as disclosed above for amplifying and detecting a target nucleic acid
- mixing said composition with a sample supposed to contain at least a first target nucleic acid
- subjecting the generated mixture to a thermocycling protocol, and
- detecting the amplification product of said target nucleic acid by means of monitoring hybridization dependent fluorescence.

In a specific embodiment, the method according to the present invention is capable of performing multiplex real time PCR. If this is the case, the composition comprises multiple hybridization probes, which are capable of hybridizing to at least two, preferably 3-4 and most preferably 3-6 different amplification targets.

In a fourth aspect, the present invention is directed to a kit consisting of

- a thermostable DNA Polymerase
- a mixture of deoxynucleoside-tri-phosphates
- a buffer
- multiple pairs of amplification primers,
- multiple hybridization probes labeled each with a different detectable fluorescent dye,

which only emits fluorescence upon binding of the probe to its target nucleic acid wherein said kit additionally comprises a Pyrophosphatase.
which preferentially is a thermostable Pyrophosphatase.

In a specific embodiment, such a kit further comprises at least a second hybridization probe labeled with a second fluorescent entity, wherein said first and second hybridization probe together constitute a pair of FRET hybridization probes.

## Detailed description of the invention

In multiplex assays fluorescence values in real-time quantification may drop below initial background fluorescence in late cycles. This effect probably occurs due to high DNA/amplicon concentrations which influence buffer conditions, reflected by a decrease of the fluorescence in the Fluorescein channel 530.

For emission of fluorescein as the FRET donor moiety in multiplex experiments, a two-phased decrease is often observed. This in turn reduces the capacity of Fluorescein to serve as a FRET donor in the fluorescence resonance energy transfer process with the consequence that Fluorescence emission of any FRET acceptor moiety are affected.

In the presence of Pyrophosphatase, the decrease in Fluorescein emission is one phased, indicating that the effect of signal decrease at late amplification cycles is at least partially compensated. This compensation can be explained by the following considerations:

During the amplification reaction, Pyrophosphate (PPi) is released upon incorporation of Nucleotide tri-Phosphates into the generated amplicon:

$$DNA_x + dNTP \leftrightarrow DNA_{x+1} + PPi$$

Increase in PPi concentration, however, results in a decrease in pH of the reaction mixture, which in turn is known to affect the stability of fluorescent dyes in general and Fluorescein in particular ("Spectrophotometric determination of pKa values for fluorescein using activity coefficient corrections", Smith, S.A., Pretorius, W.A., Water SA 28 (2002) 395-402; "Studies on Fluorescein-VII: The Fluorescence of Fluorescein as a function of pH"; Diehl, H. and Markuszewski, R., Talanta 36 (1989) 416-418).

Upon addition of thermostable Pyrophosphatase (PPase), Pyrophosphate is converted to Orthophopsphate (Pi).

$$\underset{PPi}{P_2O_7^{4-}} + H_2O \leftrightarrow \underset{Pi}{2\,HPO_4^{\,2-}}$$

In liquid solutions, the released Orthophosphate forms a balance:

$$\underset{(80\%)}{HPO_4^{\,2-}} + H_2O \leftrightarrow \underset{(20\%)}{H_2PO_4^{-}} + OH^{-}$$

Due to this balance, and specially in acidic liquids, the pH is increased and fluorescent compounds such as Fluorescein may remain stabilized.

Therefore, in a first aspect, the present disclosure is directed to the use of a Pyrophosphatase to stabilize at least a first fluorescent entity in a composition for amplification of at least a first target nucleic acid during a thermocycling procedure.

The Pyrophosphatase is preferably a thermostable Pyrophosphatase with an activity optimum above 37 °C, which is resistant to thermocycling procedures and stably retains its activity over more than 40 amplification cycles. One example of a suitable thermostable Pyrophosphatase is disclosed in EP 763 599.

The Pyrophosphatase may be added to the composition resulting in a final concentration of at least 0.005 U/µl but not more than 0.5 U/µl. Preferably, Pyrophosphatase in a final concentration of about 0.01 U/µl to 0.1 U/µl is added. As an optimum, final concentrations of about 0.04/µl Pyrophosphatase have been identified.

As theoretically explained above, addition of Pyrophosphatase during an amplification reaction results in a diminished or even completely prevented decrease of pH during the amplification procedure, especially, when different targets are amplified in a multiplex experiment. Such an effect results in stabilization of essentially any type of fluorescent entity of a flourescent probe which is present within an amplification reaction mixture, including but not limited to TaqMan probes, Molecular Beacons, Single labeled Probes and pairs of FRET Hybridization Probes. In particular, addition of Pyrophosphatase is highly advantageous for the stabilization of Fluorescein or other fluorescent compounds which may serve as a FRET donor moieties.

Moreover, addition of Pyrophosphatase is highly advantageous in case of multiplex set ups containing at least two or more different hybridization probes. In particular this holds true for multiplex FRET hybridization probes, **characterized in that** Fluorescein is used as the one and only donor component of multiple pairs of FRET Hybridization Probes.

In this regard, it needs to be noted that for other purposes, Pyrophosphatases have been used in the art of performing template dependent nucleic acid amplifications previously:

EP 763 599 discloses the use of a thermostable Pyrophosphatase as an additive in PCR to improve the overall amplification efficiency. Simililarily, DE 19612779 discloses the use of Pyrophosphatase for amplification of extraordinarily

long DNA template sequences. US 6,225,092 discloses the benefit of adding Pyrophosphatase in direct amplification sequencing reactions. US 2003/0049655 finally discloses the use of Pyrophosphate salts as an additive to PCR reaction mixes to prevent unspecific primer extensions at room temperature, as well as addition of Phosphatases to remove the respective salts subsequently. However, none of these references, and according to the best knowledge of the inventors no other prior art reference anticipates or suggests the use of Pyrophosphatase as an additive for real time PCR.

In a second aspect, the present invention is directed to a composition for amplifying and detecting a target nucleic acid consisting of

- a thermostable DNA Polymerase
- a mixture of deoxynucleoside-tri-phosphates
- a buffer
- multiple pairs of amplification primers,
- multiple hybridization probes each labeled with a different detectable fluorescent dye, which only emits fluorescence upon binding of the probe to its target nucleic acid wherein said composition additionally comprises a Pyrophosphatase.

Preferably, the Pyrophosphatase is a thermostable Pyrophosphatase. Also preferably, said Pyrophosphatase is present in a final concentration of at least 0.005 U/μl but not more than 0.5 U/μl. Highly preferred is a final concentration of about 0.01 U/μl to 0.1 U/μl with an optimum, final concentrations of about 0.04 U/μl.

The thermostable DNA polymerase may be any kind of native, recombinant and/or chemically modified DNA polymerase known in the art which is capable of performing a PCR amplification reaction. This includes for example Taq Polymerase or any other Polymerase which is capable of amplifying any kind of double stranded target DNA. Furthermore, this includes thermostable DNA Polymerases having a substantial degree of Reverse Transcriptase activity , for example DNA Polymerase from thermus thermophilus disclosed in US 5,618,711.

In particular, the DNA Polymerase may comprise a chemical midification as disclosed in US 5,677,152 and US 5,773,258. The enzymes disclosed in these references contain covalent but thermolabile modifications which render the enzyme inactive prior to the first cycle of amplification, thereby resulting in a so called Hot Start effect.

The mixture of nucleosides usually comprises dATP, dGTP, dCTP, and dTTP in about equimolar amounts sufficient to provide enough monomeric educts for the amplification reaction. Instead of dTTP, dUTP may be used. Moreover, the mixture may additionally comprise base analog d-NTPs or non-nucleoside base analog dNTPs of any kind.

In addition to an appropriate buffer, the composition may comprise further inorganic ions, salts or compounds for optimizing either the enzymatic activities of the thermostable polymerase or the preferably thermostable Pyrophosphate. Furthermore, these compounds may increase the specificity of the primer annealing. Preferably, the composition comprises $MgCl_2$ in a a concentration range between 1 and 5 mM $MgCl_2$.

The at least one pair of amplification primers is a pair of oligonucleotides the two members of which bind in opposite orientation to the target nucleic sequence. Such oligonucleotides may be prepared using conventional Phosphoramidite chemistry, which is well known in the art. This technology in addition allows the incorporration of modified or labeled nucleotides as long as appropriate Phosphoramidates are available.

The composition of the invention comprises multiple pairs of amplification primers and preferably 2, 3, 4 or5-6 pairs of amplification primers, which are capable of amplifying different target sequences that are supposed to be present in the sample that is being analyzed.

The composition according to the invention contains multiple hybridization probes each labeled with a different detectable fluorescent dye. This hybridization probe may be any kind of Hybridization probe which is used in real time PCR such as TaqMan probe, a Molecular Beacon, a Single labeled Probe. The composition according to the invention may also comprise at least one pair of FRET Hybridization Probes.

The composition according to the present invention comprises 2, 3, 4, 5-6 or even more differently labeled hybridization probes, hybridizing to different target nucleic sequences. Also within the scope of the present invention, the composition may comprise 2, 3, 4 or 5-6 pairs of FRET hybridization probes, **characterized in that** each pair of FRET hybridization probe hybridizes to a different nucleic acid sequence. Preferably, all FRET donor moieties of said multiple pairs of hybridization probes are identical. Most preferably, all donor moieties are Fluorescein.

When mixed with a sample that shall be analyzed, all inventive compositions disclosed above can be used to amplify and detect one or more target nucleic acid sequences which are supposed to be present in said sample. In this context, the present invention also covers compositions, which in addition to the ingredients present in the compositions disclosed above further comprise the respective target nucleic acid sequence or target nucleic acid sequences which are supposed to become amplified.

In another aspect, the present invention is also directed to kits for preparing a composition according to the invention.

More precisely, such a kit consisting of

- a thermostable DNA Polymerase
- a mixture of deoxynucleoside-tri-phosphates
- a buffer
- multiple pairs of amplification primers,
- multiple hybridization probes labeled each with a different detectable fluorescent dye,

which only emits fluorescence upon binding of the probe to its target nucleic acid wherein said kit additionally comprises a Pyrophosphatase.
which preferentially is a thermostable Pyrophosphatase.

The different components may be stored each individually in different vessels. Alternatively, the different components may be stored all together in one storage vessel. Also alternatively, arbitrarily selected combinations of only a subset of components (i) to (vi) may be stored together. In a prefered embodiment, components (ii), (iii), (iv), and (v) are stored together and components (i) and (vi) are each stored separately. In another prefered embodiment, (i), (ii), (iii) and (iv) and (v) are stored together.

In another embodiment, such a kit comprises multiple pairs of amplification primers and preferably 2, 3, 4 or5-6 pairs of amplification primers and respectively 2, 3, 4, or 5-6 differently labeled hybridization probes not limited to but being selected from a group consisting of TaqMan probes, Molecular Beacons and Single labeled probes.

In still another specific embodiment, such a kit further comprises at least a second hybridization probe labeled with a second fluorescent entity, wherein said first and second hybridization probe together constitute a pair of FRET hybridization probes. Furthermore, such a kit may comprise 2, 3, 4 or 5-6 pairs of amplification primers together with 2, 3, 4 or 5-6 pairs of FRET hybridization probes, **characterized in that** each pair of FRET hybridization probe hybridizes to a different nucleic acid sequence amplified by a different pair of amplification primers. Preferably, all FRET donor moieties of said multiple pairs of hybridization probes are identical. Most preferably, (at least one, several or all) donor moieties are Fluorescein.

In a very specific embodiment, a kit according to the present invention may further comprise a computer readable storage medium comprising a "color compensation files". Such a color compensation file is a means for compensating a crosstalk between different detection channels which may occur in multiplex experiments. In other words, the data obtained in each experiment are subjected to a specific mathematic operation, in order to account crosstalk between different detection channels.

In a further aspect, the present invention is also directed to a method for amplifying and detecting at least a first target nucleic acid comprising

- providing a composition for amplifying and detecting a target nucleic acid consisting of

    - a thermostable DNA Polymerase
    - a mixture of deoxynucleoside-tri-phosphates
    - a buffer
    - multiple pairs of amplification primers,
    - multiple hybridization probes each labeled with a different detectable fluorescent dye,
      which only emits fluorescence upon binding of the probe to its target nucleic acid wherein said composition additionally comprises a Pyrophosphatase.

  which preferentially is a thermostable Pyrophosphatase
- mixing said composition with a sample supposed to contain at least a first target nucleic acid
- subjecting the generated mixture to a thermocycling protocol in a real time thermocycler instrument, and
- detecting the amplification product of said target nucleic acid by means of monitoring hybridization dependent fluorescence.

For performing a method according to the present invention, all compositions and kits disclosed above may be used. In particular, the method according to the present invention is capable of performing multiplex real time PCR. If this is the case, the composition comprises multiple hybridization probes, which are capable of hybridizing to at least two, preferably 3-4 and most preferably 3-6 different amplification targets.

The new method is especially useful for multiplex PCR experiments using multiple pairs of FRET hybridization probes, especially if all FRET donor compounds are identical, for example if all FRET donor compounds are Fluorescein.

## Description of the Figures

Figures 1-5: 4plex PCR Housekeeping Genes: β2M (channel 610), PBGD (channel 640), HPRT (channel 670), G6PDH (channel 705) dotted lines without Pyrophosphatase; solid line: with 0,8 U Pyrophosphatase
Figure 1: channel 530, Fluoresceine emission
Figure 2: channel 610, LC-Red 610 emission
Figure 3: channel 640, LC-Red 640 emission
Figure 4: channel 670, Cy-5 emission
Figure 5: channel 705: LC-Red 705 emission

The following examples, references, sequence listing and figures are provided to aid the understanding of the present invention, the true scope of which is set forth in the appended claims.

**Examples**

**Example 1:**

4-Plex PCR with the FRET Hybridization probe format in a LightCycler 2.0 instrument

For amplification and detection of the 4 different housekeeping genes h-PBGD, h-β2-M, h-G6PDH, and hHPRT in a Light Cycler 2.0 Instrument (Roche Applied Science Cat. No. 3 531 414 201), primers and probes with sequences according to Roche Applied Science Cat. Nos . 3 146 073, 3 146 081, 3 261 883, and 3 261 891 were used. Amplification was performed in the presence or absence of 0.8 units thermostable Pyrophosphatase (Calbiochem Cat. No.: 405822)
First, 10x detection mixes were prepared:

β2M (β2 Microglobulin)

| | final concentration |
|---|---|
| H2O | |
| β2M forward Primer | 5 μmol |
| β2M reverse Primer | 5 μmol |
| β2M Fluorescein Probe | 2 μmol |
| β2M LC Red610 Probe | 2 μmol |

HPRT (Hypoxanthine Phosphoribosyl Transferase)

| | final concentration |
|---|---|
| H2O | |
| HPRT forward Primer | 3 μmol |
| HPRT reverse Primer | 3 μmol |
| HPRT Fluorescein Probe | 2 μmol |
| HPRT Cy-5 Probe | 2 μmol |

G6PDH (Glucose-6-Phosphate-Dehydrogenase)

| | final concentration |
|---|---|
| H2O | |
| G6PDH forward Primer | 5 μmol |
| G6PDH reverse Primer | 5 μmol |
| G6PDH Fluorescein Probe | 2 μmol |
| G6PDH LC Red705 Probe | 2 μmol |

PBGD (Porphobilinogen Deaminase)

| | final concentration |
|---|---|
| H2O | |
| PBGD forward Primer | 3 μmol |
| PBGD reverse Primer | 5 μmol |
| PBGD Fluorescein Probe | 2 μmol |
| PBGD LightCycler Red 640 Probe | 2 μmol |

Primers and probes had been synthesized according to standard protocols of Phosphporamidate chemistry using Fluorescein-CPG (Roche Applied Science Cat. No. 3 138 187, LC-Red-610 (Roche Applied Science Cat. 3 561 488), LC-Red 640 (Roche Applied Science Cat. No. 2 015 161), LC-Red-705 (Roche Applied Science Cat. No. 2 157 594) and Cy-5-NHS-ester (Amersham Cat. No. 27179901) or Cy-5 Phosphoramidate (Amersham Cat. No. PA 15100).

For each capillary in a total volume of 20 μl, 4 at 5x Master Mix (Multiplex Master Roche Applied Science Cat. No. 04 340 019 001) were mixed with 0.8 μl 25 mM $MgCl_2$ and 2 μl 10x Detection Mix each, 4 μl human cDNA containing about $10^5$ copies of a respective DNA reverse transcribed from RNA standards of Roche Applied Science Cat Nos. 3 146 073, 3 146 081, 3 261883, and 3 261 891). In each second aliquot, 0,8 μl thermostable Pyrophosphatase ( 1 U/μl, Calbiochem Cat. No.: 405822) were added resulting in a final concentration of 0,04 U/μl).

The respective pipetting scheme was as follows:

| | per capillary without Pyrophosphatase | per capillary with Pyrophosphatase |
|---|---|---|
| 5 x LightCycler Multiplex DNA Master HybProbe (Cat. No. 04 340 019 001) | 4 μl | 4 μl |
| $MgCl_2$ (25 mM) | 0.8 μl | 0.8 μl |
| 10 x Detection Mix β2M | 2 μl | 2 μl |
| 10 x Detection Mix HPRT | 2 μl | 2 μl |
| 10 x Detection Mix G6PDH | 2 μl | 2 μl |
| 10 x Detection Mix PBGD | 2 μl | 2 μl |
| cDNA $10^5$ copies of a respective DNA reverse transcribed from RNA standards of Roche Applied Science Cat Nos. 3 146 073, 3 146 081, 3 261883, and 3 261 891 | 4 μl | 4 μl |
| Pyrophosphatase ( 1 U/μl, Calbiochem Cat. No.: 405822) | - | 0.8 μl |
| $H_2O$ | 3.2 μl | 2.4 μl |
| total | 20 μl | 20 μl |

Thermocycling and detection in real time was done according to the following protocol:

| | | | | | |
|---|---|---|---|---|---|
| Denaturation | 1x | 95°C | 600 s | 20 °C/s | - |
| Amplification | 45x | 95°C | 10 s | 20 °C/s | - |
| | | 55°C | 10 s | 20 °C/s | Single |
| | | 72°C | 10 s | 20 °C/s | - |
| Cooling | 1x | 40°C | 30 s | 20 °C/s | - |

Fluorescence emissions from the different detection channels is shown in figures 1-5, **characterized in that** complete lines represent a sample containing Pyrophosphatase and dotted lines represent a sample containing no Pyrophosphatase.

Emission of fluorescein as the FRET donor moiety of all 4 different pairs of FRET hybridization probes is shown in Fig. 1. As it can be seen in the figure, a two-phased decrease can be measured, while in the presence of 0.8 U Pyrophosphatase, the decrease is one phased. Thus Pyrophosphatase is able to stabilze at least partially Fluorescein during late amplification cycles.

Emission of the different FRET acceptor moieties indicative for amplification and detection of the 4 different housekeeping genes (Fig. 2 channel 610, h-β2-M), Fig. 3, channel 640 (PBGD), Fig. 4, channel 670 (HPRT), and Fig. 5, channel 705 (G6PDH)) in the presence or absence of Pyrophosphatase is shown in figures 2-5 over 45 cycles of amplification.

As it can be seen from the figures, in a sample without Pyrophosphatase, fluorescence signaling in all 4 channels peaked at about 35 amplification cycles and dropped off subsequently. This effect was completely compensated in a sample comprising additional 0.8 U Pyrophospatase for FRET donor probes labeled with LC-Red 610 (fig. 2), LC-Red 640 (fig. 3) and LC-Red 705 (fig. 5), and substantially compensated for probes labeled with Cy-5 (fig. 4). Thus, addition of Phosphatase is able to stabilize signaling of the different FRET acceptor moieties at late amplification cycles, probably due to stabilization of the FRET donor Fluorescein.

**Example 2:**

**3-Plex PCR with the FRET Hybridization probe format in a LightCycler 2.0 instrument**

Under conditions basically identical to example 1, a 3-plex Real time PCR experiment was performed in order to amplify three different sequences from $1x10^6$ copies of plasmid DNA each. Thermostable Pyrophosphatase from Roche Applied Science (Cat. No. 1 721 992) was used. PCR was performed with appropriate primers and probes for detecting Factor II DNA using LC-Red 640 as fluorescent acceptor moiety and the two hHFE isoforms HFE845 using Cy-5 as fluorescent acceptor moiety and HFE187, using LC-Red 705 as fluorescent acceptor moiety. Probes carrying a 5' acceptor moiety were blocked at their respective 3' end with a terminal Phosphate moiety.

For these three targets, the following primers and probes were used:

**Prothrombin (Factor II) Accession No. AF478696**

| |
|---|
| **FII forward Primer**<br>5'cca atc ccg tga aag aat tat 3'<br>SEQ. ID. NO: 1 |
| **FII reversed Primer**<br>5'agg tgg tgg att ctt aag tc 3'<br>SEQ. ID. NO: 2 |
| **FII Fluorescein Probe**<br>5'gag cat tgt ggc tcg ctg ag-Fluos 3'<br>SEQ. ID. NO: 3 |
| **FII LC Red 640 Probe**<br>5'LC Red 640-cac ttt tat tgg gaa cca tag ttt tag aaa casc aa-Phos 3'<br>SEQ. ID. NO: 4 |

**HFE 187 Accession No. Z92910**

| |
|---|
| **HFE 187 forward Primer**<br>5'gcc tca gag cag gac ctt gg 3'<br>SEQ. ID. NO: 5 |
| **HFE 187 reversed Primer** |

(continued)

| |
|---|
| 5'cag ctg ttt cct tca aga tgc 3'<br>SEQ. ID. NO: 6 |
| **HFE 187 Fluorescein Probe**<br>5'ctt gaa att cta ctg gaa acc cat gga gtt egg ggc tcc-Fluos 3'<br>SEQ. ID. NO: 7 |
| **HFE 187 LC Red 705 Probe**<br>5'LC Red 705-cac ggc gac tct cat cat cat aga aca cga aca-Phos 3'<br>SEQ. ID. NO: 8 |

**HFE 845 Accession No. Z92910**

| |
|---|
| **HFE 845 forward Primer**<br>5'-tgg caa ggg taa aca gat cc-3'<br>SEQ. ID. NO: 9 |
| **HFE 845 reversed Primer**<br>5'-ctc agg cac tcc tct caa cc-3'<br>SEQ. ID. NO: 10 |
| **HFE 845 Fluorescein Probe**<br>5'-aga tat acg tac cag gtg gag-Fluos3'<br>SEQ. ID. NO: 11 |
| **HFE 845 Cy5 Probe**<br>5'Cy5-ccc agg cct gga tca gcc cct cat tgt gat ctg gg- Phos 3'<br>SEQ. ID. NO: 12 |

Also in this experiment, dropping of fluorescent signaling at late amplification cycles could be compensated by the addition of 0.8 units Pyrophosphatase completely with respect to the LC-Red 640 and LC-Red 705 emission, and substantially with respect to the Cy-5 emission.

## List of References

Bernard, P.S., et al., Anal. Biochem. 255 (1998) 101-107 DE 19612779
Diehl, H., and Markuszewski, R., Talanta 36 (1989) 416-418 EP 763 599
Matthews, J.A., and Kricka, L.J., Anal. Biochem. 169 (1988) 1-25
Smith, S.A., Pretorius, W.A., Water SA 28 (2002) 395-402
US 2003/0049655
US 5,118,801
US 5,210,015
US 5,487,972
US 5,538,848
US 5,618,711
US 5,677,152
US 5,773,258
US 5,804,375
US 6,174,670
US 6,225,092
WO 02/14555
WO 97/46707
WO 97/46712
WO 97/46714

## Claims

**1.** A composition for amplifying and detecting a target nucleic acid consisting of

- a thermostable DNA Polymerase
- a mixture of deoxynucleoside-tri-phosphates
- a buffer
- multiple pairs of amplification primers,
- multiple hybridization probes each labeled with a different detectable fluorescent dye,

which only emits fluorescence upon binding of the probe to its target nucleic acid wherein said composition additionally comprises a Pyrophosphatase.

**2.** A composition according to claim 1, wherein said Pyrophosphatase is a thermostable Pyrophsophatase.

**3.** A composition consisting of a composition according to claims 1-2, and a template nucleic acid.

**4.** A kit consisting of

- a thermostable DNA Polymerase
- a mixture of deoxynucleoside-tri-phosphates
- a buffer
- multiple pairs of amplification primers,
- multiple hybridization probes labeled each with a different detectable fluorescent dye,

which only emits fluorescence upon binding of the probe to its target nucleic acid wherein said kit additionally

comprises a Pyrophosphatase.

**5.** A kit consisting of a kit according to claim 4, and a computer readable storage medium comprising a color compensation file.

**6.** A method for amplifying and detecting at least a first target nucleic acid comprising

- providing a composition according to claims 1-2,
- mixing said composition with a sample supposed to contain at least a first target nucleic acid
- subjecting the generated mixture to a thermocycling protocol
- detecting the amplification product of said target nucleic acid by means of monitoring hybridization dependent fluorescence in real time.

**7.** A method according to claim 6, **characterized in that** said multiple hybridization probes, are capable of hybridizing to at least two, preferably 3-6 and most preferably 3-4 different amplification products.

**Patentansprüche**

**1.** Zusammensetzung zum Amplifizieren und Nachweisen einer Zielnukleinsäure, bestehend aus

- einer thermostabilen DNA-Polymerase,
- einem Gemisch von Desoxynukleosidtriphosphaten,
- einem Puffer,
- mehreren Amplifikationsprimer-Paaren,
- mehreren jeweils mit einem unterschiedlichen nachweisbaren Fluoreszenzfarbstoff markierten Hybridisierungssonden,

wobei der Fluoreszenzfarbstoff nur bei Bindung der Sonde an ihre Zielnukleinsäure Fluoreszenz emittiert, wobei die Zusammensetzung zusätzlich eine Pyrophosphatase umfasst.

**2.** Zusammensetzung nach Anspruch 1, wobei es sich bei der Pyrophosphatase um eine thermostabile Pyrophosphatase handelt.

**3.** Zusammensetzung, bestehend aus einer Zusammensetzung nach Anspruch 1-2 und einer Matrizennukleinsäure.

**4.** Kit, bestehend aus

- einer thermostabilen DNA-Polymerase,
- einem Gemisch von Desoxynukleosidtriphosphaten,
- einem Puffer,
- mehreren Amplifikationsprimer-Paaren,
- mehreren jeweils mit einem unterschiedlichen nachweisbaren Fluoreszenzfarbstoff markierten Hybridisierungssonden,

wobei der Fluoreszenzfarbstoff nur bei Bindung der Sonde an ihre Zielnukleinsäure Fluoreszenz emittiert, wobei der Kit zusätzlich eine Pyrophosphatase umfasst.

**5.** Kit, bestehend aus einem Kit nach Anspruch 4 und einem eine Farbabgleichsdatei umfassenden computerlesbaren Datenträger.

**6.** Verfahren zum Amplifizieren und Nachweisen wenigstens einer Zielnukleinsäure, umfassend

- Bereitstellen einer Zusammensetzung nach Anspruch 1-2,
- Mischen der Zusammensetzung mit einer vermutlich wenigstens eine erste Zielnukleinsäure enthaltenden Probe,
- Ausführen eines Thermocycling-Protokolls mit dem erzeugten Gemisch,

- Nachweisen des Amplifikationsprodukts der Zielnukleinsäure mittels Verfolgen der hybridisierungsabhängigen Fluoreszenz in Echtzeit.

**7.** Verfahren nach Anspruch 6, **dadurch gekennzeichnet, dass** die mehreren Hybridisierungssonden in der Lage sind, an wenigstens zwei, vorzugsweise 3-6 und am stärksten bevorzugt 3-4 unterschiedliche Amplifikationsprodukte zu hybridisieren.

## Revendications

**1.** Composition d'amplification et de détection d'un acide nucléique cible constituée de

- une ADN polymérase thermostable
- un mélange de désoxynucléoside-tri-phosphates
- un tampon
- paires multiples d'amorces d'amplification,
- sondes d'hybridation multiples chacune marquée avec un colorant fluorescent détectable différent,

qui émet seulement de la fluorescence à l'issue de la liaison de la sonde à son acide nucléique cible dans laquelle ladite composition comprend de manière supplémentaire une pyrophosphatase.

**2.** Composition selon la revendication 1, dans laquelle ladite pyrophosphatase est une pyrophosphatase thermostable.

**3.** Composition constituée d'une composition selon les revendications 1 à 2, et d'un acide nucléique du type matrice.

**4.** Kit constitué de

- une ADN polymérase thermostable
- un mélange de désoxynucléoside-tri-phosphates
- un tampon
- paires multiples d'amorces d'amplification,
- sondes d'hybridation multiples chacune marquée avec un colorant fluorescent détectable différent,

qui émet seulement de la fluorescence à l'issue de la liaison de la sonde à son acide nucléique cible dans lequel ledit kit comprend de manière supplémentaire une pyrophosphatase.

**5.** Kit constitué d'un kit selon la revendication 4, et d'un support lisible par ordinateur comprenant un fichier de compensation de couleur.

**6.** Procédé d'amplification et de détection d'au moins un premier acide nucléique cible comprenant

- la fourniture d'une composition selon les revendications 1 à 2,
- le mélange de ladite composition avec un échantillon supposé contenir au moins un premier acide nucléique cible
- la soumission du mélange généré à un protocole de thermocyclage
- la détection du produit d'amplification dudit acide nucléique cible au moyen de la surveillance de la fluorescence fonction de l'hybridation en temps réel.

**7.** Procédé selon la revendication 6, **caractérisé en ce que** lesdites sondes d'hybridation multiples, sont capables de s'hybrider à au moins deux, de préférence 3 à 6 et de manière préférée entre toutes 3 à 4 produits différents d'amplification.

Fig. 1

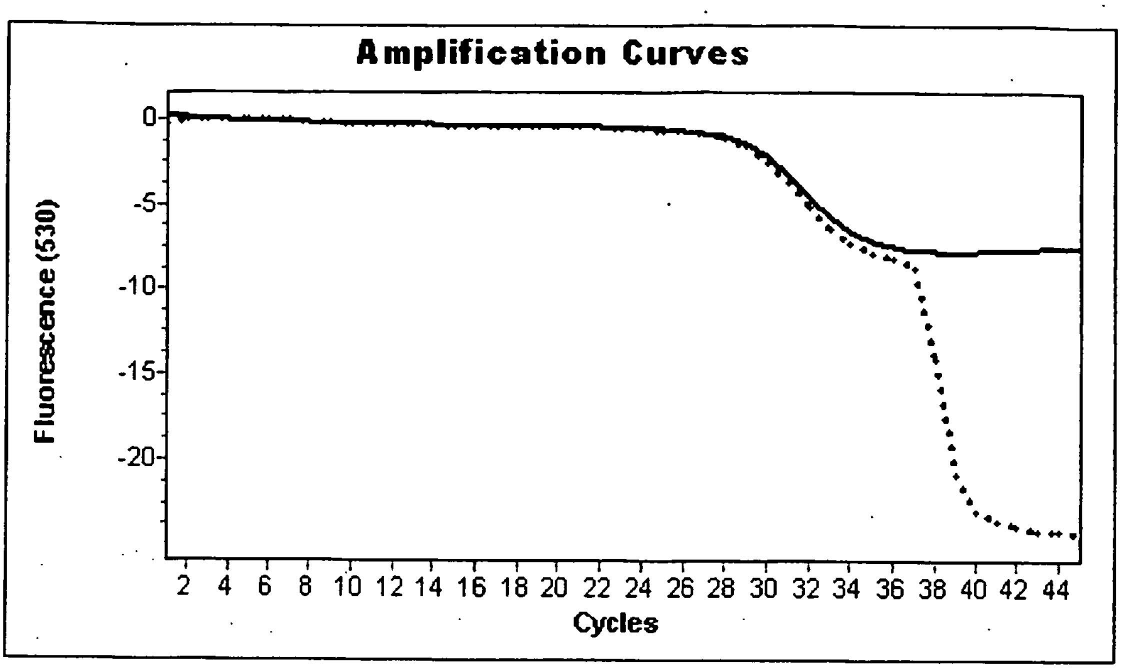
Amplification Curves
Fluorescence (530)
0
-5
-10
-15
-20
2 4 6 8 10 12 14 16 18 20 22 24 26 28 30 32 34 36 38 40 42 44
Cycles

Fig. 2

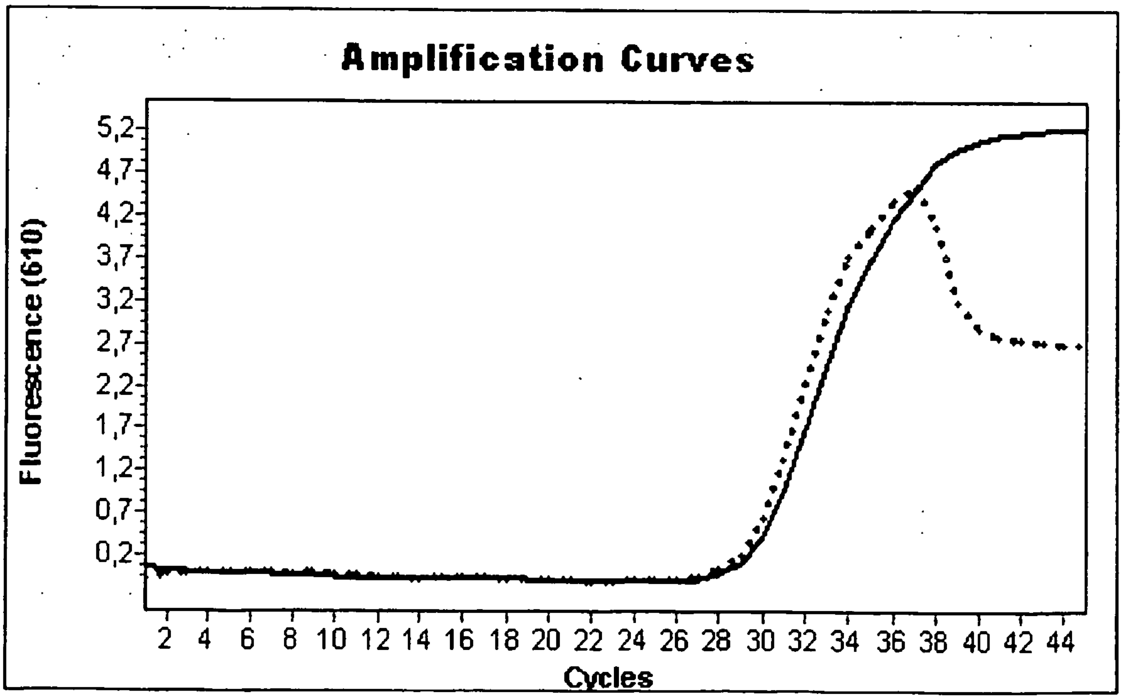
Amplification Curves
Fluorescence (610)
5,2
4,7
4,2
3,7
3,2
2,7
2,2
1,7
1,2
0,7
0,2
2 4 6 8 10 12 14 16 18 20 22 24 26 28 30 32 34 36 38 40 42 44
Cycles

Fig. 3

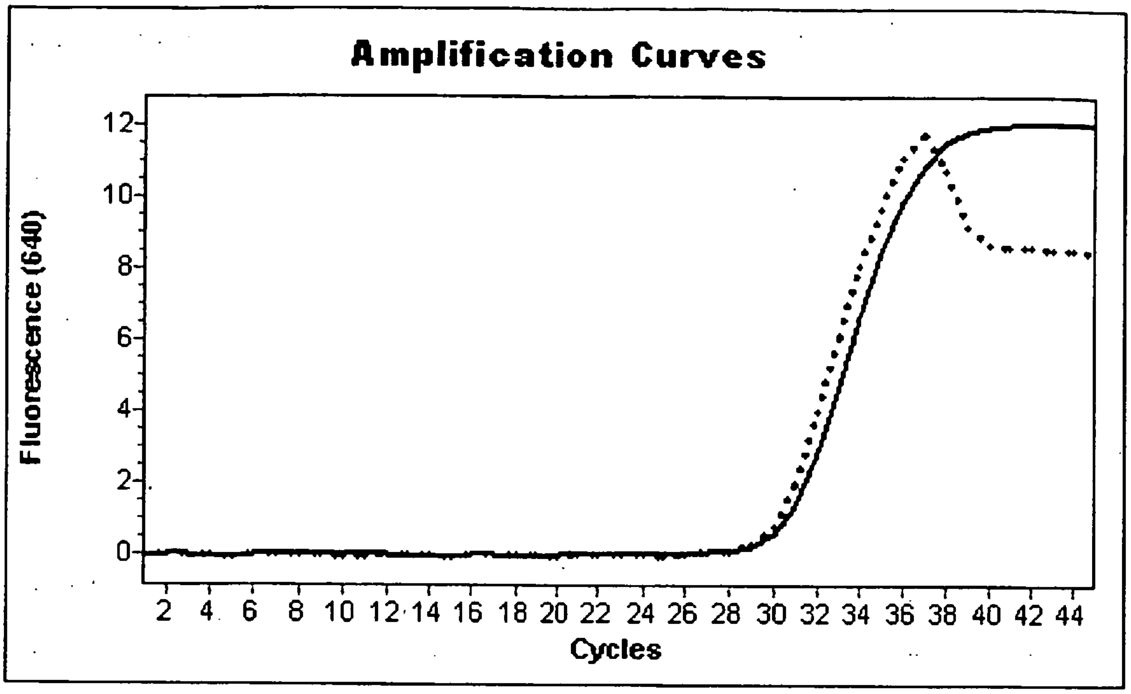
Amplification Curves
Fluorescence (640)
0
2
4
6
8
10
12
2 4 6 8 10 12 14 16 18 20 22 24 26 28 30 32 34 36 38 40 42 44
Cycles

Fig. 4

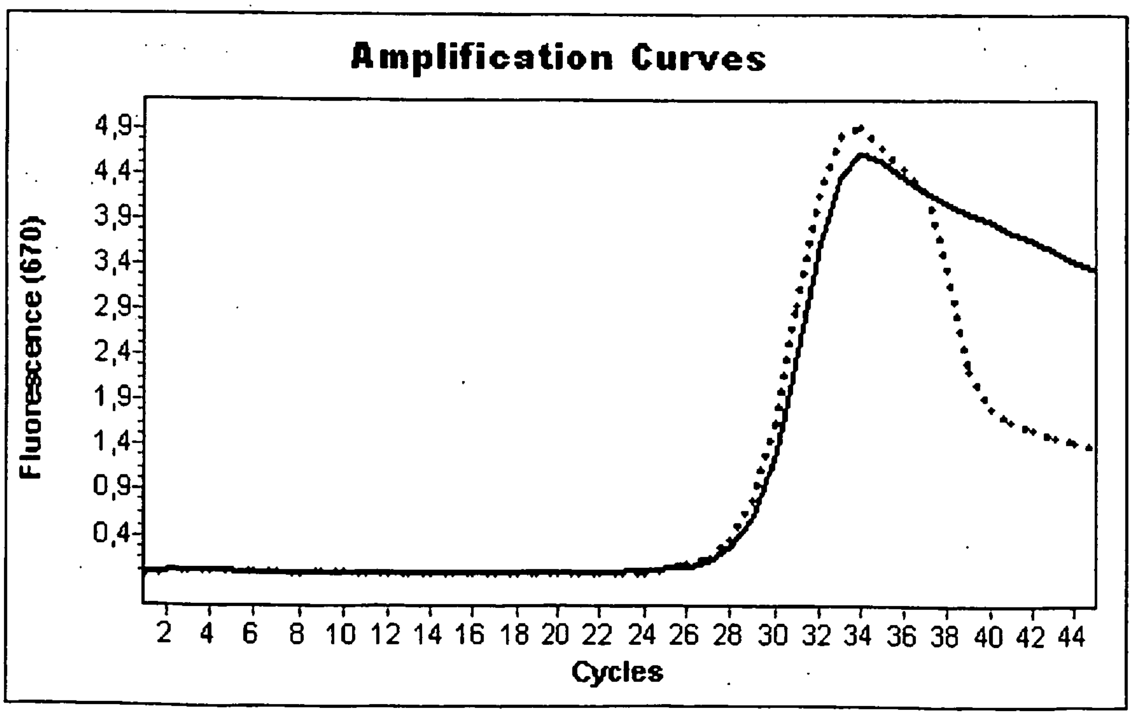
Amplification Curves
Fluorescence (670)
0,4
0,9
1,4
1,9
2,4
2,9
3,4
3,9
4,4
4,9
2 4 6 8 10 12 14 16 18 20 22 24 26 28 30 32 34 36 38 40 42 44
Cycles

Fig. 5

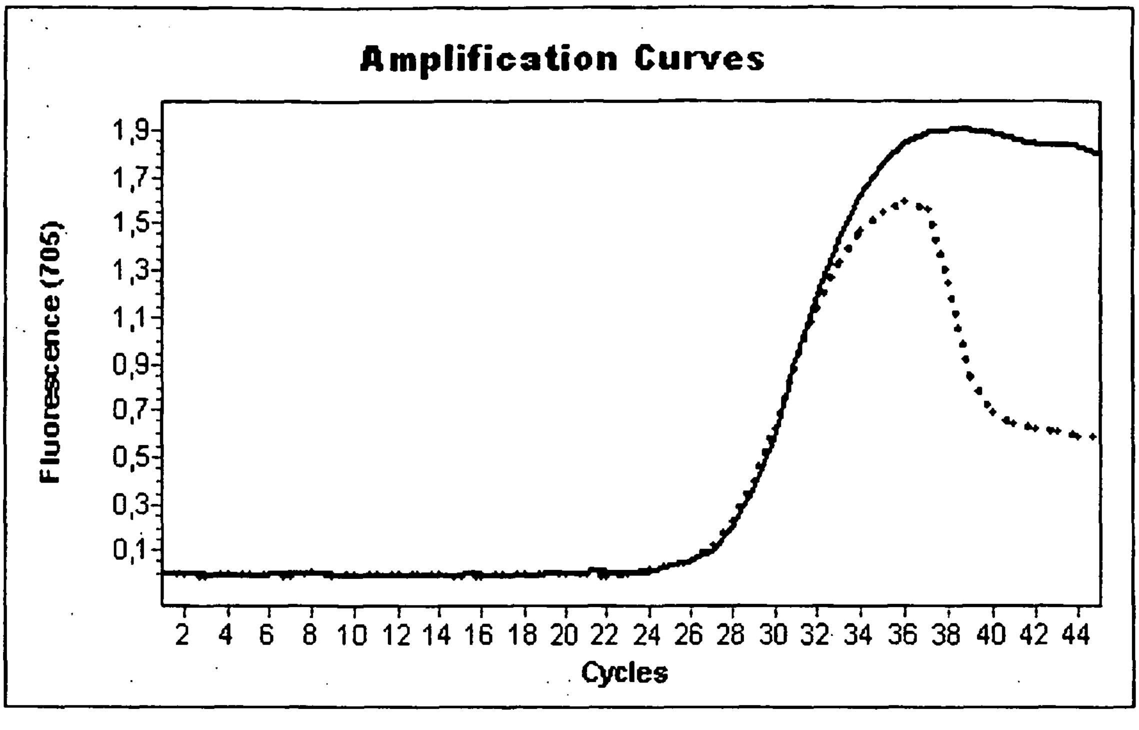
Amplification Curves
Fluorescence (705)
1,9
1,7
1,5
1,3
1,1
0,9
0,7
0,5
0,3
0,1
2 4 6 8 10 12 14 16 18 20 22 24 26 28 30 32 34 36 38 40 42 44
Cycles

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6174670 B
- US 5210015 A
- US 5538848 A
- US 5487972 A
- US 5804375 A
- US 5118801 A
- WO 0214555 A
- WO 9746707 A
- WO 9746712 A
- WO 9746714 A
- EP 763599 A
- DE 19612779
- US 6225092 B
- US 20030049655 A
- US 5618711 A
- US 5677152 A
- US 5773258 A
- EP 04021399 A


### Non-patent literature cited in the description


- **Matthews, J.A. ; Kricka, L.J.** *Anal. Biochem.,* 1988, vol. 169, 1-25
- **Bernard, P.S. et al.** *Anal. Biochem.,* 1998, vol. 255, 101-107
- **Smith, S.A. ; Pretorius, W.A. ; Water SA.** *Spectrophotometric determination of pKa values for fluorescein using activity coefficient corrections,* 2002, vol. 28, 395-402
- **Diehl, H. ; Markuszewski, R.** *Studies on Fluorescein-VII: The Fluorescence of Fluorescein as a function of pH,* 1989, vol. 36, 416-418
- **Diehl, H. ; Markuszewski, R.** *Talanta,* 1989, vol. 36, 416-418
- **Smith, S.A. ; Pretorius, W.A. ; Water SA.** *ANAL. BIOCHEM.,* 2002, vol. 28, 395-402

SEQUENCE LISTING

<110> F. Hoffmann-La Roche AG

<120> Real time PCR with the addition of Pyrophosphatase

<130> 22722 AF

<150> EP 04021399
<151> 2004-09-09

<160> 12

<170> PatentIn version 3.2

<210> 1
<211> 21
<212> DNA
<213> Artificial

<220>
<223> FII forward Primer

<400> 1
ccaatcccgt gaaagaatta t    21

<210> 2
<211> 20
<212> DNA
<213> Artificial

<220>
<223> FII reversed Primer

<400> 2
aggtggtgga ttcttaagtc    20

<210> 3
<211> 20
<212> DNA
<213> Artificial

<220>
<223> FII Fluorescein Probe

<400> 3
gagcattgtg gctcgctgag    20

<210> 4
<211> 36
<212> DNA
<213> Artificial

<220>
<223> FII LC Red 640 Probe

<400> 4
cacttttatt gggaaccata gttttagaaa cascaa 36

<210> 5
<211> 20
<212> DNA
<213> Artificial

<220>
<223> HFE 187 forward Primer

<400> 5
gcctcagagc aggaccttgg 20

<210> 6
<211> 21
<212> DNA
<213> Artificial

<220>
<223> HFE 187 reversed Primer

<400> 6
cagctgtttc cttcaagatg c 21

<210> 7
<211> 39
<212> DNA
<213> Artificial

<220>
<223> HFE 187 Fluorescein Probe

<400> 7
cttgaaattc tactggaaac ccatggagtt cggggctcc 39

<210> 8
<211> 33
<212> DNA
<213> Artificial

<220>
<223> HFE 187 LC Red 705 Probe

<400> 8
cacggcgact ctcatcatca tagaacacga aca 33

<210> 9
<211> 20
<212> DNA
<213> Artificial

<220>
<223> HFE 845 forward Primer

<400> 9
tggcaagggt aaacagatcc 20

<210> 10
<211> 20
<212> DNA
<213> Artificial

<220>
<223> HFE 845 reversed Primer

<400> 10
ctcaggcact cctctcaacc 20

<210> 11
<211> 21
<212> DNA
<213> Artificial

<220>
<223> HFE 845 Fluorescein Probe

<400> 11
agatatacgt accaggtgga g 21

<210> 12
<211> 35
<212> DNA
<213> Artificial

<220>
<223> HFE 845 Cy5 Probe

<400> 12
cccaggcctg gatcagcccc tcattgtgat ctggg 35